# EUROPEAN PATENT APPLICATION

(11) **EP 4 109 492 A2**
(43) Date of publication of application: **28.12.2022**
(21) Application number: 22175665.3
(22) Date of filing: 26.05.2022
(51) Int. Cl.: H01J 61/35, A61L 2/10, H01J 61/40, H01J 65/04

(54) **ULTRAVIOLET LAMP TUBE**

(30) Priority: 22.06.2021 CN 202110689201; 10.02.2022 CN 202220273286 U
(71) Applicant: Langsim Optoelectronic Technologies (Guangdong) Limited, Nanhai, Foshan Guangdong (CN)
(72) Inventor: Li, Jiawei, FOSHAN (CN)
(74) Representative: Díaz de Bustamante y Terminel, Isidro

(57) **Abstract**

This application discloses an ultraviolet lamp tube comprising: an outer tube 1 and an inner tube 2, each installed with an electrode 4,3, wherein the outer wall of the outer tube is coated with plated coatings 12 and 11 for filtering waves to enable emission of a single-wavelength light from the light source. Alternatively, coating 12 is a single-wavelength filter coating and coating 11 is a reflective coating to enable emission of a single-wavelength light with a high luminous efficiency. Through the method of plated coatings, the UV lamps disclosed have achieved increased beam angle, improved light output power and luminous efficiency.

## Description

An ultraviolet lamp tube.

### CROSS-REFERENCE TO RELATED APPLICATION

This application claims priority to Chinese Patent Application No. 202110689201.7, filed on June 22, 2021, and Chinese Patent Application No. 202220273286.0, filed on February 10, 2022, with the China National Intellectual Property Administration, the disclosures of both of which are incorporated herein by reference in their entirety.

### FIELD OF THE DISCLOSURE

This disclosure relates to the technical field of ultraviolet lamps, in particular, to an ultraviolet lamp tube with a high luminous efficiency.

### BACKGROUND OF THE DISCLOSURE

Ultraviolet light wavelengths are divided into long waves, medium waves and short waves. Among them, short waves can be used for disinfection and sterilization, while medium waves can cause certain harm to human body. The existing ultraviolet lamps designed for strong output of short waves are often accompanied by the medium waves. Some of these ultraviolet light tubes use a light filter to filter the wavelength bands harmful to the human body; however, these structures still have the defects of small beam angle and non-monochromatic light source, thus reducing the luminous efficiency of the entire ultraviolet lamp. To this end, we provide a UV lamp tube to solve the above-mentioned problems.

### SUMMARY OF THE DISCLOSURE

Aiming at the defects of the existing ultraviolet lamps in the above-mentioned background technonolgies, such as small beam angle, non-monochromatic light source that cannot emit light in a single wavelength band, and poor luminous efficiency, the present disclosure provides a novel ultraviolet lamp tube to overcome these defects.

In one aspect, the present disclosure provides an ultraviolet lamp tube comprising an outer tube and an inner tube, the inner tube installed inside the outer tube; an outer electrode installed on the tube wall of the outer tube; an inner electrode installed inside of the inner tube; the outer wall of the outer tube coated with plated coating A and plated coating B, respectively.

In one embodiment, sometimes preferred, the wavelengths of the wavelength bands that can be transmitted by the coating film A and the coating film B are different.

In one embodiment, sometimes preferred, the coating film A can transmit a single usable wavelength band.

In one embodiment, sometimes preferred, the coating film B can transmit other wavelengths that the coating film A cannot transmit, and at the same time reflect a single wavelength band that can pass through the coating film A to the coating film A and transmit it out.

In one embodiment, sometimes preferred, both the plated coating A and the plated coating B are light-filtering coatings.

In one embodiment, sometimes preferred, the plated coating A is a light-filtering coating, which can selectively transmit a desired wavelength band; the coating B can reflect the desired wavelength band back to the coating A on the outer tube and transmit it out.

In one aspect, the present disclosure provides an ultraviolet lamp tube, comprising an inner tube and an outer tube arranged coaxially; an inner electrode installed in the inner tube; an outer electrode embedded on the outer tube; wherein the outer wall of the outer tube is coated with coating A; the outer wall of the outer tube is fill-coated with a reflective coating; the coating A and the reflective coating are arranged opposite to each other. In one embodiment, the coating A is a coating that can transmit a single wavelength band; and the coating A can only transmit a wavelength band of a single wavelength out of the outer tube.

In another embodiment, the reflective coating reflects the clutter waves that cannot pass through the coating A to the inner tube, and transmits them to the inner electrode through the inner tube, wherein the clutter waves will be absorbed by the inner tube wall and the electrode; at the same time, the reflective coating reflects the light to the coating A side and transmits the useful light wave out of the outer tube.

In one embodiment, the reflective coating is a barium sulfate reflective coating layer.

In another embodiment, the reflective coating is a titanium dioxide reflective coating.

In another aspect, the present disclosure provides a device comprising an ultraviolet lamp tube according to any embodiment disclosed herein, for example, while not intended to be limiting, the novel ultraviolet lamp tube can be used in a disinfection device for antibacterial and/or antiviral applications, preferably in a space occupied by humans and/or animals, including but not limited to domestic pets or farm-raised animals, such as cats, dogs, monkeys, horses, pigs, cattles, poultry, or the like.

Other aspects or advantages of the disclosure will be better appreciated in view of the following drawings, detailed description, examples, and claims.

### BRIEF DESCRIPTION OF THE DRAWINGS

FIG. 1 is the structural schematic diagram of an exemplary ultraviolet lamp tube, in which: 1-outer tube; 2-inner tube; 3-inner electrode; and 4-outer electrode.
FIG. 2 is a cross-sectional view of the ultraviolet lamp tube, in which: 1-outer tube; 2-inner tube; 11-plated coating B; and 12-plated coating A.
FIG. 3 is a schematic structural diagram of an exemplary ultraviolet lamp tube, in which: 1-inner tube; 2-outer tube; 3-inner electrode; 4-outer electrode; 5-coating A; and 6-reflective coating.

### DETAILED DESCRIPTION OF CERTAIN EMBODIMENTS

The present invention will now be described in further details with reference to the accompanying drawings. These drawings are all simplified schematic diagrams, and only illustrate the basic structure of the present invention in a schematic manner, so they only show the structures related to the present invention.

In the present disclosure, it should be noted that the orientation or positional relationships indicated by the terms "inside", "outside", "between", or the like, are based on the orientation or positional relationships shown in the accompanying drawings. They only serve for the convenience of describing the present invention and simplifying the description, rather than indicate or imply that the referred device or element must have a specific orientation, or be constructed and operated in a specific orientation, so they should not be construed to be limiting on the present invention.

In addition, unless otherwise expressly specified and defined, the terms "install", "wind", and "connect" should be understood in a broad sense, for example, it can be a fixed connection, a detachable connection, or an integral connection; it can be a mechanical connection or an electrical connection; it can be a direct connection or an indirect connection through an intermediate medium; and it can be internal communication between two components of a unit. For those of ordinary skill in the art, the specific meanings of the above terms in the present disclosure can be understood under the specific circumstances.

### EXAMPLES

### Example 1

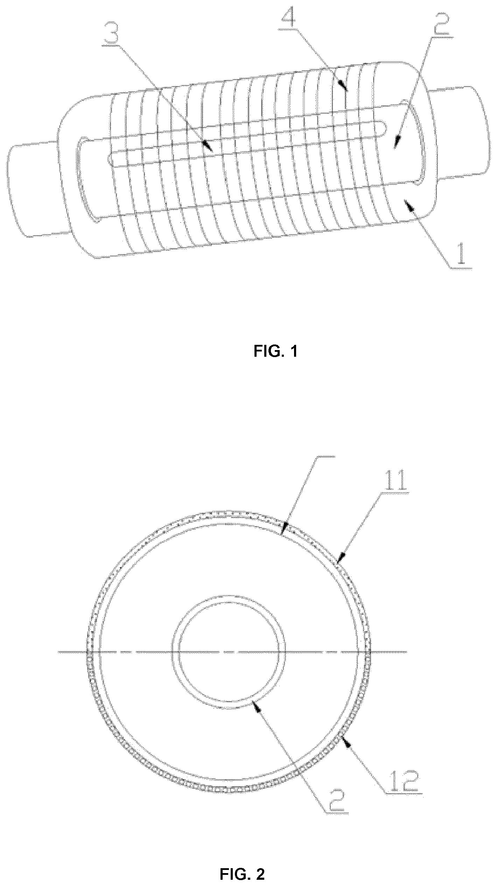

As shown in FIGs. 1-2, the present disclosure provides an ultraviolet lamp tube, comprising an outer tube 1 and an inner tube 2; the inner tube 2 installed inside the outer tube 1; an outer electrode 4 installed on the tube wall of the outer tube 1; an inner electrode 3 installed in the inner tube 2. The outer wall of the outer tube 1 is coated with coating A12 and coating B11, respectively.

The wavelengths of the wavelength bands that can be transmitted by the coating film A12 and the coating film B11 are different; the coating film A can transmit a single usable wavelength band; the coating film B can transmit other wavelengths that the coating film A cannot transmit, and at the same time reflect a single wavelength band that can pass through the coating film A to the coating film A and transmit it.

The present invention uses the method of plated coating for light filtering, which can ensure the monochromaticity of light output from the light source. Secondly, in comparison with use of light filters for filtering waves, the use of plated coatings can enhance the output power of ultraviolet light and improve the photoelectric conversion efficiency. At the same time, the area ratio of coating A12 and coating B11 can be adjusted to effectively increase the beam angle of the UV lamp; this method can effectively improve the luminous efficiency of the UV lamp; for example, the coating A can transmit a single usable wavelength band, such as a single wavelength band of 207 nm, 222 nm, 250 nm, or 308 nm, or the like, while the coating B can transmit the clutter wavelength bands that the coating A cannot transmit to be absorbed by the box body, thereby effectively reduce the harm to the human body.

### Example 2

As shown in FIGs. 1-2, the present disclosure provides an ultraviolet lamp tube, comprising an outer tube 1 and an inner tube 2; the inner tube 2 is arranged inside the outer tube 1; an outer electrode 4 is arranged on the tube wall of the outer tube 1; an inner electrode 3 is arranged in the inner tube 2; the outer wall of the outer tube 1 is coated with coating A12 and coating B11, respectively; whereas, the coating A12 is a filter coating, and the coating A can selectively transmit a desired single wavelength band, such as a single wavelength band of 207 nm, 222 nm, 250 nm, or 308 nm, or the like; the coating B11 is mirror coating that can reflect any wavelength band back to the outer tube 1, that is, the mirror coating can reflect a single wavelength band projected to the coating B11 to the coating A12 and pass through the coating A12, thereby enhancing the luminous efficiency of the UV lamp. The clutter waves with wavelengths harmful to the human body are projected to the box body along the end of the UV lamp and absorbed by the box body, thereby effectively reducing the harm to people.

### Example 3

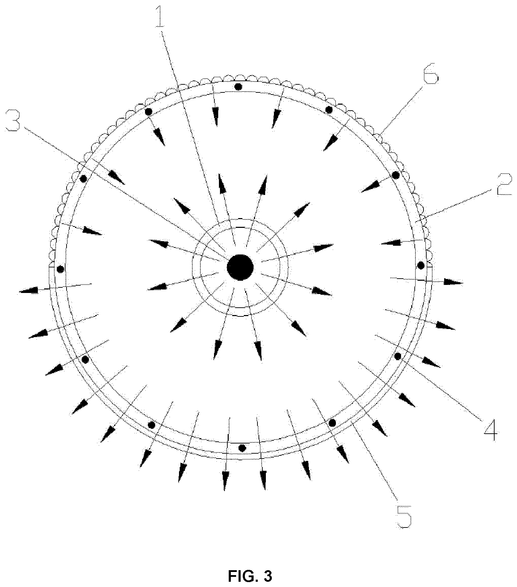

As shown in FIG. 3, the disclosure provides an ultraviolet lamp tube, comprising an inner tube 1 and an outer tube 2 arranged coaxially; inside the inner tube 1 is installed an inner electrode 3; the outer tube 2 is embedded with an outer electrode 4; the outer wall of the outer tube 2 is plated with coating A 5; wherein, the coating A 5 is a coating that can transmit a single wavelength band; the coating A 5 can only transmit a wavelength band of a single wavelength out of the outer tube 2; the outer wall of the outer tube 2 is also fill-coated with a reflective coating 6; and the coating A 5 and the reflective coating 6 are arranged opposite to each other. The reflective coating 6 can be a barium sulfate reflective coating or a titanium dioxide reflective coating; wherein, the reflective coating 6 reflects the clutter waves that cannot pass through the coating A 5 to the inner tube 1, which are transmitted to the inner electrode 3 through the inner tube 1 and absorbed by the inner tube wall and the inner electrode 3; and at the same time, the reflective coating 6 reflects the light to the coating A 5 side and transmits the useful light waves out of the outer tube 1.

The ultraviolet lamp tube adopts a coating method for light reflection to ensure the single wavelength light output emitted from a light source and to enhance the luminous efficiency of the ultraviolet lamp, in which the outer wall of the outer tube 2 is coated with coating A 5 and fill-coated with a reflective coating 6, wherein the coating A 5 is a coating film that can transmit a single wavelength band and can transmit the useful single wavelength band out of the outer tube 2. The reflective coating 6 can be a barium sulfate reflective coating or a titanium dioxide reflective coating, or the like, since barium sulfate or titanium dioxide is non-conductive and chemically stable and has high reflectivity to ultraviolet light.

Advantages: first, the UV lamp tubes disclosed herein contain plated coatings for light filtering, which can ensure the monochromaticity of light output from the light source. Second, in comparison with light filters for filtering waves, the use of plated coatings can enhance the output power of ultraviolet light and improve the photoelectric conversion efficiency. At the same time, the area ratio of coating A and coating B can be set to effectively increase the beam angle of the UV lamp; thus this method can effectively improve the luminous efficiency of the UV lamp.

Specifically, the coating A can transmit a single usable wavelength band, such as a single wavelength band of 207 nm, 222 nm, 250 nm, or 308 nm, or the like, while the coating B can transmit clutter wavelength bands that the plated coating A cannot transmit to be absorbed by the box body, thereby effectively reducing the harm to the human body.

In another ultraviolet lamp tube disclosed, the coating method for light reflections can also ensure the light output efficiency of the light source and the single wavelength of the light output, through a plated coating A and a fill-coated reflective coating on the outer wall of the outer tube, wherein the coating A is a coating that can transmit a single wavelength band and transmit the useful single wavelength band to the outside of the outer tube. Use of a barium sulfate reflective coating or titanium dioxide reflective coating is advantageous because they are non-conductive and chemically stable and have a high reflectivity to UV light. In the description of this specification, the reference terms such as "one embodiment, "some embodiments", "exemplary embodiment", "example", "specific example", or "some examples", or the like, mean that a particularfeature, structure, material, or characteristic described in such embodiment or an example is included in at least one embodiment or illustrative example of the present invention. In this application, schematic representations of the above terms do not necessarily refer to the same embodiment or example. Furthermore, the particular features, structures, materials or characteristics described may be combined in a suitable manner in any one or more embodiments or examples.

The above has shown and described the basic principles and major features and advantages of the present invention, and it will be apparent to those skilled in the art that the present invention is not limited to the details of the above-described exemplary embodiments, and that without departing from the spirit or essential characteristics of the present invention, the present invention can be implemented in other specific forms. Therefore, the embodiments are to be regarded in all respects as illustrative and non-limiting, and the scope of the invention is defined by the appended claims rather than the foregoing description, and it is therefore intended to embrace within the present invention all changes or modifications that come within the meaning and range or equivalents of the claims. Any reference symbols or numbers to the drawings in the claims shall not be construed as limiting the involved claims. In addition, it should be understood that although this invention is described in terms of embodiments, it is not the case that each embodiment only includes an independent technical solution, and this description in the specification is only for the purpose of clarity, and those skilled in the art should take the specification as a whole, since the technical solutions in each embodiment can also be appropriately combined to form other embodiments that can be understood by those skilled in the art.

## Claims

1. An ultraviolet lamp tube, comprising: an outer tube and an inner tube; the inner tube installed inside of the outer tube; an outer electrode installed on tube wall of the outer tube; and an inner electrode installed inside of the inner tube; wherein the outer wall of the outer tube is coated with a plated coating film A and a plated coating film B, respectively.

2. The ultraviolet lamp tube of claim 1, wherein the wavelengths of the wave bands that can be transmitted by the coating film A and the coating film B are different.

3. The ultraviolet lamp tube of claim 2, wherein the coating film A can transmit usable single wavelength band.

4. The ultraviolet lamp tube of claim 3, wherein the coating film B can transmit other wavelengths that the coating film A cannot transmit, and at the same time reflect a single wavelength band transmitted by the coating film A back to the coating film A for transmitting out.

5. The ultraviolet lamp tube of claim 1, wherein the coating film A and the coating film B are both plated light-filtering coatings.

6. The ultraviolet lamp tube of claim 1, wherein the coating film A is a filter coating that can selectively transmit a desired wavelength band; and wherein the coating film B is a filter coating that can reflect the desired wavelength band back to the plated coating film A on the outer tube and transmit it out.

7. An ultraviolet lamp tube, comprising an inner tube and an outer tube arranged coaxially; an inner electrode installed in the inner tube; and an outer electrode embedded on the outer tube; the outer wall of the outer tube is plated with coating A, and the outer wall of the outer tube is fill-coated with a reflective coating; and wherein the coating A and the reflective coating are arranged opposite to each other.

8. The ultraviolet lamp tube of claim 7, wherein the coating A is a coating that can transmit a single wavelength band; the coating A can only transmit a single wavelength band to the outside of the outer tube.

9. The ultraviolet lamp tube of claim 8, wherein the reflective coating reflects clutter waves that cannot pass through the coating A to the inner tube and transmits them to the inner electrode through the inner tube; wherein the clutter waves are absorbed by the inner tube wall and electrode; and wherein, at the same time, the reflective coating reflects the light to the coating A side and transmits the useful light waves out of the outer tube.

10. The ultraviolet lamp tube of claim 9, wherein the reflective coating is a barium sulfate reflective coating or a titanium dioxide reflective coating.

11. A device comprising an ultraviolet lamp tube of claim 1.

12. The device of claim 11, which is a disinfection device.

13. A device comprising an ultraviolet lamp tube of claim 7.

14. The device of claim 13, which is a disinfection device.
